# EUROPEAN PATENT APPLICATION

(11) **EP 4 685 371 A2**
(43) Date of publication of application: **28.01.2026**
(21) Application number: 25190433.0
(22) Date of filing: 18.07.2025
(51) Int. Cl.: F16K 11/074, B01L 3/00, G01N 35/10

(54) **FLOW PATH SELECTION VALVE, FLUIDIC SYSTEM, AND METHOD FOR CONTROLLING FLUID FLOW**

(30) Priority: 22.07.2024 CN 202410987299
(71) Applicant: GeneMind Biosciences Company Limited, Shenzhen City, Guangdong 518023 (CN)
(72) Inventor: MA, Minghui, 518023 Shenzhen City (CN); HAN, Weichun, 518023 Shenzhen City (CN); WU, Ping, 518023 Shenzhen City (CN)
(74) Representative: Schiweck Weinzierl Koch Patentanwälte Partnerschaft mbB

(57) **Abstract**

The present disclosure provides a flow path selection valve (10), a fluidic system, and a method for controlling fluid flow. The flow path selection valve (10) is configured to switch between a first valve position and a second valve position, and the flow path selection valve (10) is provided with a common port (11), a plurality of first ports (12), a plurality of second ports (13), a first communication groove (14), and second communication grooves (15). In a case that the flow path selection valve (10) is in the first valve position, the plurality of first ports (12) are in communication with the common port (11) through the first communication groove (14); in a case that the flow path selection valve is in the second valve position, the first ports (12) are in communication with the second ports (13) through the second communication grooves (15) in a one-to-one correspondence. In this way, the control over the switching of fluid flow paths can be achieved, the number of three-way valves in the fluidic system can be reduced, and the structure of the fluidic system can be simplified, such that the cost of the fluidic system is lowered, and the sequencing cost is further reduced. In addition, different fluid inlet modes can be selected, which enhances the flexibility of fluid loading, thereby improving the sequencing quality and sequencing efficiency.

## Description

### TECHNICAL FIELD

The present disclosure relates to the field of gene sequencing, and in particular, to a flow path selection valve, a fluidic system, and a method for controlling fluid flow.

### BACKGROUND

Gene sequencing technology refers to the technical means of acquiring the base sequence of DNA or RNA by assays. The current dominant sequencing technology is high-throughput sequencing. In a sequencing platform that achieves high-throughput sequencing based on sequencing by synthesis, the general gene sequencing process includes: fixing a nucleic acid sample under test on a flow cell, for example, by hybridization; forming a nucleic acid molecule cluster on the nucleic acid sample under test by using PCR amplification; adding sequencing reagents (e.g., bases with fluorophores, polymerases, primers, and the like) to the flow cell through a fluidic system; bonding the bases with the fluorophores to the base on the nucleic acid sample under test according to the base complementary pairing principle; exciting the fluorophores by an optical imaging system to generate fluorescence; collecting the fluorescence for forming an image; and performing base calling on the image, so as to achieve base sequence determination of the nucleic acid sample under test.

In the related art, the complex structure of the fluidic system and the single fluid inlet mode result in a slow speed of the sample under test and reagents entering the flow cell and a prolonged fluid inlet time, thereby reducing the fluid inlet efficiency and sequencing efficiency.

### SUMMARY

The present disclosure provides a flow path selection valve, a fluidic system, and a method for controlling fluid flow.

The embodiments of the present application provide a flow path selection valve. The flow path selection valve is configured to switch between a first valve position and a second valve position, and the flow path selection valve is provided with a common port, a plurality of first ports, a plurality of second ports, a first communication groove, and second communication grooves. In a case that the flow path selection valve is in the first valve position, the plurality of first ports are in communication with the common port through the first communication groove; in a case that the flow path selection valve is in the second valve position, the first ports are in communication with the second ports through the second communication grooves in a one-to-one correspondence.

In this way, the flow path selection valve can allow the first ports to be in selective combination with the common port and the second ports. With this configuration, the control over the switching of fluid flow paths can be achieved, the number of three-way valves in the fluidic system can be reduced, and the structure of the fluidic system can be simplified, such that the cost of the fluidic system is reduced, and thus the sequencing cost is reduced. In addition, the fluid may flow from the common ports to the plurality of first ports via the first communication groove, or may flow from the plurality of first ports to the corresponding second ports via the plurality of second communication grooves. Therefore, different fluid inlet modes can be selected, which enhances the flexibility of fluid loading, thereby improving the sequencing quality and sequencing efficiency.

In some embodiments, the first communication groove includes a main communication groove and a plurality of branch communication grooves. The plurality of branch communication grooves are all in communication with the main communication groove, and the plurality of branch communication grooves are arranged in a one-to-one correspondence with the plurality of first ports; the plurality of first ports are in communication with the common port through the main communication groove and the plurality of branch communication grooves.

In some embodiments, the first communication groove includes a main communication groove and a plurality of sets of branch communication grooves. The plurality of sets of branch communication grooves are in communication with the main communication groove, the plurality of sets of branch communication grooves include a plurality of branch communication grooves, and the plurality of branch communication grooves are arranged in a one-to-one correspondence with the plurality of first ports; the plurality of first ports are in communication with the common port through the main communication groove and the plurality of branch communication grooves.

In some embodiments, the plurality of sets of branch communication grooves include a first set of branch communication grooves and a second set of branch communication grooves. The first set of branch communication grooves is in communication with the main communication groove through a first flow path; the second set of branch communication grooves is in communication with the main communication groove through a second flow path.

In some embodiments, the main communication groove is provided with a fluid inlet end, each of the branch communication grooves is provided with a fluid outlet end, the fluid inlet end is in communication with the common port, and the fluid outlet ends are in communication with the first ports.

In some embodiments, a plurality of second communication grooves are provided. Each of the second communication grooves has two ends, where one end of each of the second communication grooves is in communication with one of the first ports, and the other end is in communication with one of the second ports.

In some embodiments, the flow path selection valve includes a stator and a rotor disposed opposite to the stator. The stator is provided with the common port, the plurality of first ports, and the plurality of second ports. The rotor is provided with the first communication groove and the second communication grooves.

In some embodiments, the number of the common ports is plural. In the case that the flow path selection valve is in the first valve position, each of the common ports is in communication with the plurality of first ports through the first communication groove.

The embodiments of the present application provide a fluidic system. The fluidic system includes a flow cell, a flow path selection valve, and a power assembly. The flow cell includes a plurality of fluid channels, and the fluid channels are configured to carry a sample under test and/or a reagent. The flow path selection valve is disposed upstream of the flow cell, and a plurality of second ports of the flow path selection valve are in communication with and arranged in a one-to-one correspondence with the plurality of fluid channels. The power assembly is configured to provide power to drive the sample under test and/or the reagent to enter the fluid channels via the flow path selection valve.

In some embodiments, the fluidic system includes a first reservoir. The first reservoir is in communication with the common port of the flow path selection valve, and the first reservoir is configured to store the sample under test and/or the reagent.

In some embodiments, the power assembly includes a first pump group and a second pump group. The first pump group is disposed upstream of the flow cell and is in communication with the first ports of the flow path selection valve, the first pump group is in selective communication with the flow path selection valve and the first reservoir, and the first pump group is configured to aspirate the sample under test and/or the reagent in a negative pressure-driven manner, and drive, in a positive pressure-driven manner, the sample under test and/or the reagent to enter the fluid channels. The second pump group is disposed downstream of the flow cell, and the second pump group is configured to drive, in a negative pressure-driven manner, the sample under test and/or the reagent aspirated by the first pump group to enter the fluid channels.

In some embodiments, the fluidic system includes a plurality of first buffer regions. Each of the first buffer regions is in communication with one of the first ports and the first pump group. In the case that the flow path selection valve is in the first valve position, the first pump group is configured to aspirate a single sample under test and/or reagent from the first reservoir in a negative pressure-driven manner, and pump the single sample under test and/or the reagent to each of the first buffer regions.

In some embodiments, in the case that the flow path selection valve is in the second valve position, the first pump group is configured to pump the sample under test and/or the reagent in each of the first buffer regions to the corresponding fluid channel in a positive pressure-driven manner; and/or the second pump group is configured to pump the sample under test and/or the reagent in each of the first buffer regions to the corresponding fluid channel in a negative pressure-driven manner.

In some embodiments, the fluidic system includes a plurality of first switching valves. The first switching valves are disposed between the first pump group and the flow path selection valve and are configured to control the communication and disconnection between the first pump group and the flow path selection valve.

In some embodiments, the fluidic system includes a plurality of second buffer regions and a plurality of second reservoirs. Each of the second buffer regions is in communication with the first pump group and one second switching valve, and the first pump group is configured to aspirate a plurality of samples under test from the second reservoirs in a negative pressure-driven manner, and pump each of the samples under test to one of the second buffer regions corresponding thereto. In some embodiments, in the case that the flow path selection valve is in the second valve position, the first pump group is configured to pump the sample under test in each of the second buffer regions to the corresponding fluid channel in a positive pressure-driven manner; and/or the second pump group is configured to pump the sample under test in each of the second buffer regions to the corresponding fluid channel in a negative pressure-driven manner.

In some embodiments, the fluidic system includes a plurality of second switching valves. Each of the second switching valves is disposed between the second reservoir and the second buffer region corresponding thereto and is configured to control the communication and disconnection between the second reservoir and the second buffer region.

In some embodiments, the fluidic system includes a rotary valve. The rotary valve is in communication with the first reservoir and the common port of the flow path selection valve.

The embodiments of the present application provide a method for controlling fluid flow for use in the fluidic system according to any one of the above embodiments. The method includes:
allowing the power assembly to drive, in both a positive pressure-driven manner and a negative pressure-driven manner, the sample under test and/or the reagent to enter the fluid channel via the flow path selection valve.

In some embodiments, allowing the power assembly to drive, in both a positive pressure-driven manner and a negative pressure-driven manner, the sample under test and/or the reagent to enter the fluid channel via the flow path selection valve includes:
allowing the first pump group to aspirate the sample under test and/or the reagent in a negative pressure-driven manner; and
allowing the first pump group and the second pump group to simultaneously drive, in a positive pressure-driven manner and a negative pressure-driven manner, respectively, the sample under test and/or the reagent aspirated by the first pump group to enter the fluid channel via the flow path selection valve.

In some embodiments, the method includes:
in the case that the flow path selection valve is in the first valve position, allowing the first pump group to aspirate a single sample under test and/or reagent from the first reservoir in a negative pressure-driven manner, and pumping the single sample under test and/or reagent to each of the first buffer regions.

In some embodiments, the method includes:
in the case that the flow path selection valve is in the second valve position, allowing the first pump group and the second pump group to simultaneously pump, in a positive pressure-driven manner and a negative pressure-driven manner, respectively, the sample under test and/or the reagent in each of the first buffer regions to the corresponding fluid channel via the flow path selection valve.

In some embodiments, the method includes:
allowing the first pump group to aspirate a plurality of samples under test from the second reservoirs in a negative pressure-driven manner, and pump each of the samples under test to one of the second buffer regions corresponding thereto.

In some embodiments, the method includes:
in the case that the flow path selection valve is in the second valve position, allowing the first pump group and the second pump group to simultaneously pump, in a positive pressure-driven manner and a negative pressure-driven manner, respectively, the sample under test in each of the second buffer regions to the corresponding fluid channel via the flow path selection valve.

The embodiments of the present application provide a computer storage medium. A computer program, when run by a processor, causes the processor to implement the method according to any one of the above embodiments.

Additional aspects and advantages of the present disclosure will be partially provided in the following description, will partially become apparent from the following description, or will be learned through the practice of the present disclosure.

### BRIEF DESCRIPTION OF THE DRAWINGS

The aforementioned and/or additional aspects and advantages of the present disclosure will become apparent and easily understood from the description of the embodiments with reference to the following drawings, in which:
FIG. 1 is a schematic structural view of a flow path selection valve according to an embodiment of the present disclosure;
FIG. 2 is a schematic structural view of a flow path selection valve according to an embodiment of the present disclosure;
FIG. 3 is a schematic structural view of a flow path selection valve according to an embodiment of the present disclosure;
FIG. 4 is a schematic structural view of a fluidic system according to an embodiment of the present disclosure;
FIG. 5 is a schematic flowchart of a control method according to an embodiment of the present disclosure.

Description of the reference numerals: 100, fluidic system; 10, flow path selection valve; 11, common port; 12, first port; 13, second port; 14, first communication groove; 141, main communication groove; 142, branch communication groove; 143, first flow path; 144, second flow path; 15, second communication groove; 16, stator; 17, rotor; 20, flow cell; 21, fluid channel; 30, power assembly; 31, first pump group; 32, second pump group; 33, first single pump; 34, second single pump; 40, first reservoir; 41, second reservoir; 50, first buffer region; 51, second buffer region; 60, first switching valve; 61, second switching valve; 70, rotary valve; and 80, waste fluid bottle.

### DETAILED DESCRIPTION

The embodiments of the present disclosure are described in detail below, and the examples of the embodiments are shown in the drawings, throughout which identical or similar reference numerals represent identical or similar elements or elements having identical or similar functionality. The embodiments described below with reference to the drawings are exemplary and are merely intended to illustrate the present disclosure, and should not be construed as limiting the present disclosure.

In the description of the present disclosure, it should be understood that orientational or positional relationships indicated by terms such as "central", "longitudinal", "transverse", "length", "width", "thickness", "upper", "lower", "front", "rear", "left", "right", "vertical", "horizontal", "top", "bottom", "inner", "outer", "clockwise", or "counterclockwise", are those shown on the basis of the drawings, and are merely intended to facilitate and simplify the description rather than indicate or imply that the indicated apparatus or element must have a specific orientation and be configured and operated according to the specific orientation. Such relationships should not be construed as limiting the present disclosure. In addition, the terms "first" and "second" are used herein for descriptive purposes only and should not be construed as indicating or implying relative importance or implicitly indicating the number of technical features described. Therefore, features defined with "first" and "second" may explicitly or implicitly include one or more of the features. In the description of the present disclosure, unless otherwise clearly and specifically defined, the term "plurality" means two or more.

In the description of the present disclosure, it should be noted that unless otherwise clearly specified and defined, the terms "mount", "link", and "connect" should be interpreted in their broad sense. For example, the connection may be a fixed connection, detachable connection, or integral connection; a mechanical connection, electric connection, or communicative connection; or a direct connection, indirect connection through an intermediate, internal communication of two elements, or interaction between two elements. For those of ordinary skill in the art, the specific meanings of the aforementioned terms in the present disclosure can be interpreted according to specific conditions.

In the present disclosure, unless otherwise clearly specified and defined, a first feature being "above" or "below" a second feature may include that the first and second features are in direct contact and that the first and second features are not in direct contact but are in contact via an additional feature therebetween. Moreover, a first feature being "on", "over", and "above" a second feature includes that the first feature is right above or obliquely above the second feature, or simply means that the first feature is at a vertically higher position than the second feature. A first feature being "under", "beneath", and "below" a second feature includes that the first feature is right below or obliquely below the second feature, or simply means that the first feature is at a vertically lower position than the second feature.

The following disclosure provides many different embodiments or examples for implementing different structures of the present disclosure. To simplify the disclosure of the present application, the components and settings of specific examples are described below. Certainly, the examples are merely exemplary and are not intended to limit the present disclosure. In addition, reference numerals and/or characters may be repeatedly used in different examples in the present disclosure for simplicity and clarity rather than to indicate the relationship between various embodiments and/or settings discussed. In addition, the present disclosure provides examples of various specific processes and materials, but those of ordinary skill in the art will recognize the application of other processes and/or the use of other materials.

As used herein, "sequencing" refers to nucleic acid sequence determination, synonymous with "nucleic acid sequencing" or "gene sequencing", which refers to the determination of the order of bases in the primary structure of a nucleic acid molecule. This can be achieved by sequencing by synthesis (SBS), sequencing by ligation (SBL), sequencing by hybridization (SBH), or the like. The sequencing by synthesis includes, in addition to the generally understood SBS (typical ILLUMINA/Solexa technology) in which incorporation of a nucleotide into a sample under test is catalyzed by using a polymerase (synthesis reaction) and a corresponding reaction signal is detected to identify the type of the incorporated nucleotide, sequencing similar to SBS, in which a nucleotide is controllably introduced or ligated to a sample under test by using a polymerase or a non-polymerase, and a corresponding signal is directly or indirectly detected to determine the type of the ligated nucleotide.

The sequencing may include DNA sequencing and/or RNA sequencing, which includes long fragment sequencing and/or short fragment sequencing (the long fragment and short fragment are defined relatively; for example, nucleic acid molecules longer than 1 Kb, 2 Kb, 5 Kb, or 10 Kb may be referred to as long fragments, and nucleic acid molecules shorter than 1 Kb or 800 bp may be referred to as short fragments); and may include double-end sequencing, single-end sequencing, paired-end sequencing, and/or the like, where the double-end sequencing or the paired-end sequencing may refer to the reading of any two segments or two portions of the same nucleic acid molecule that are not completely overlapping.

The sequencing may be performed through a sequencing platform. According to the embodiments of the present application, the optional sequencing platforms include, but are not limited to Illumina's Hiseq, Miseq, Nextseq, and Novaseq sequencing platforms, Thermo Fisher/Life Technologies' Ion Torrent platform, BGI's BGISEQ and MGISEQ/DNBSEQ platforms, and single-molecule sequencing platforms. The sequencing method may be selected from single-read sequencing, double-end sequencing, or sequencing methods supported by the selected automated sequencing platform.

The sequencing generally includes: library preparation, PCR amplification (optional), sequencing, and data analysis. The sequences assayed and read by sequencing are referred to as sequencing sequences, also referred to as reads.

In some examples, the sequencing sequences or the reads are acquired by performing a plurality of rounds of sequencing using sequencing by synthesis. For example, a sample under test is allowed to be in contact with a polymerase and an altered nucleotide and be subjected to conditions suitable for a polymerization reaction, the altered nucleotide is allowed to be controllably incorporated into the sample under test or a single base extension is controllably achieved, a corresponding reaction signal is detected, the type of the nucleotide incorporated into the sample under test in the reaction is determined based on the signal, and a plurality of controllable single base extensions and detections of corresponding signals are performed to assay the types of the nucleotides or bases incorporated into the sample under test in a plurality of reactions or a plurality of rounds of reactions based on the reaction signal information, thereby assaying and reading a portion of the sequence of the sample under test.

The sample under test, also referred to as a template or a template under test, may be a single molecule without amplification, or a molecular cluster or a long strand containing a plurality of identical polynucleotide molecules after amplification, such as a clonal cluster or a DNA nanoball (DNB) formed by bridge amplification or rolling circle amplification used in mainstream sequencing platforms. The sample under test may be in the form of a single strand, a double strand, and/or a complex hybridized with the probe or primer.

The corresponding reaction signals may be, for example, fluorescence signals, and may be converted into image data generated by collecting the fluorescence signals, and thus, the image data are processed and analyzed to assay the nucleotide incorporated into the sample under test in each reaction or each round of reaction, so as to determine a portion of the base sequence of the sample under test.

Specifically, in some examples, sequencing is achieved based on surface fluorescence imaging assay. The sample under test is ligated to the solid-phase surface, for example, nucleotides can be altered to have or bind fluorescent labels and cleavable inhibiting groups that can prevent other nucleotides from being polymerized and ligated to the next position of the sample under test (the altered nucleotides are also referred to as reversible terminators), and excitation is performed after each polymerization reaction or single base extension reaction to make the fluorescent labels emit light; the light-emitting signals are collected to acquire an image of the sample under test where the single base extension reaction occurs at the designated surface position. Next, the inhibiting groups, the fluorescent labels, and the like are removed to perform the next or the next round of polymerization reaction and signal acquisition (photographing). A plurality of or a plurality of rounds of polymerization-photographing-excision are repeated to acquire image set information on the nucleotide ligated to the sample under test in association with each single base extension reaction.

It can be understood that the sample under test at the designated position on the surface undergoes a polymerization reaction to emit fluorescence, which generally appears as a bright point or spot with an intensity higher than the background signal at the corresponding position of the image collected in the round of reaction. Therefore, according to the image set which includes the information on the bright spot corresponding to specific chemical features (the sample under test in which the polymerization reaction occurs), whether the sample under test at the designated position undergoes a polymerization reaction can be determined. The type of nucleotide that has been polymerized and ligated to the sample under test in the polymerization reaction can be assayed through the conjunction of the corresponding relationship between the preset distinguishable fluorescent light-emitting signal and the type of the nucleotide, such that at least a portion of the sequence of the sample under test can be determined to acquire the read.

It should be noted that the nucleotide includes ribonucleic acid or deoxyribonucleic acid, and includes natural nucleotides, derivatives thereof, or altered versions thereof (also referred to as altered nucleotides, modified nucleotides, or the like). As used herein, the nucleotide is sometimes referred to by the base it contains, which can be understood by those skilled in the art based on conventional knowledge and/or context.

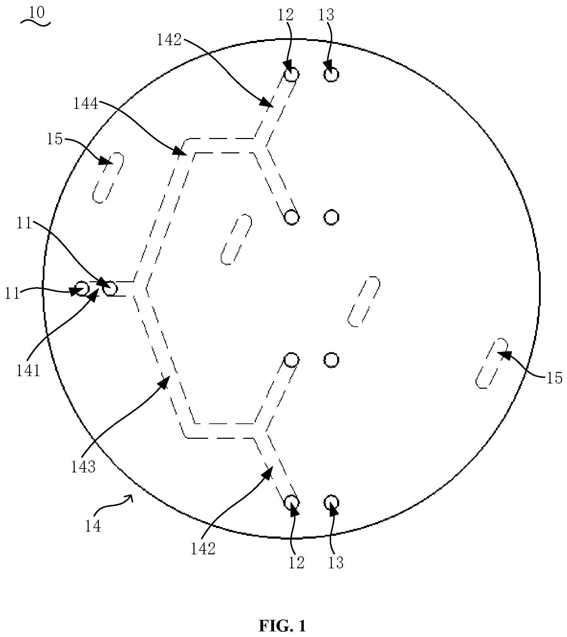

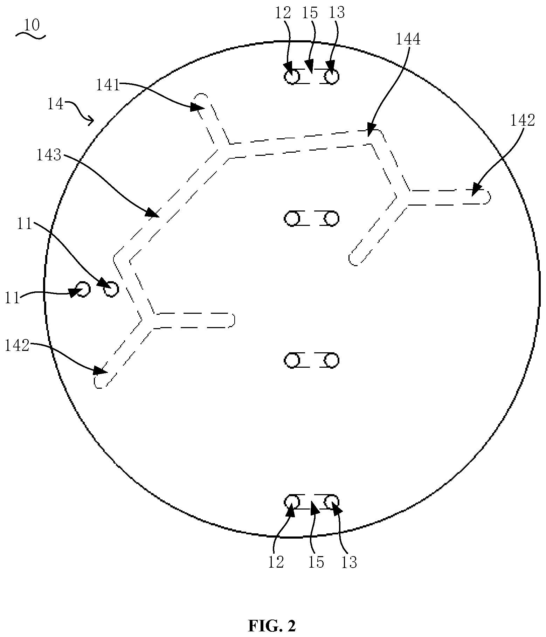

Referring to FIGs. 1 and 2, the embodiments of the present application provide a flow path selection valve 10. The flow path selection valve 10 is configured to switch between a first valve position and a second valve position, and the flow path selection valve 10 is provided with a common port 11, a plurality of first ports 12, a plurality of second ports 13, a first communication groove 14, and second communication grooves 15. In the case that the flow path selection valve 10 is in the first valve position, the plurality of first ports 12 are in communication with the common port 11 through the first communication groove 14; in the case that the flow path selection valve 10 is in the second valve position, the first ports 12 are in communication with the second ports 13 through the second communication grooves 15 in a one-to-one correspondence.

In this way, the flow path selection valve 10 can allow the first ports 12 to be in selective communication with the common ports 11 and the second ports 13. With this configuration, the control over the switching of fluid flow paths can be achieved, the number of three-way valves in the fluidic system 100 can be reduced, and the structure of the fluidic system 100 can be simplified, such that the cost of the fluidic system 100 is reduced, and thus the sequencing cost is reduced. In addition, the fluid may flow from the common ports 11 to the plurality of first ports 12 via the first communication groove 14, or may flow from the plurality of first ports 12 to the corresponding second ports 13 via the plurality of second communication grooves 15. Therefore, different fluid inlet modes can be selected, which enhances the flexibility of fluid loading, thereby improving the sequencing quality and sequencing efficiency.

Specifically, the common port 11 may serve as a fluid inlet or a fluid outlet of the flow path selection valve 10. In other words, the fluid may enter or exit the flow path selection valve 10 from the common port 11, thereby achieving flow diversion or flow confluence. In the present application, the common port 11 serves as the fluid inlet of the flow path selection valve 10, and the fluid may enter the flow path selection valve 10 from the common port 11. The common port 11 may be of a regular shape, such as a circular shape or a polygonal shape, or of an irregular shape. The shape of each common port 11 may be the same or different. In an embodiment of the present application, to facilitate the formation and manufacturing of the common port 11 and/or the connection thereof with a common tube, the common port 11 is of a circular shape.

The first port 12 and the second port 13 may serve as a fluid inlet or a fluid outlet of the flow path selection valve 10. The number of the first port 12 may be the same as the number of the second port 13. For example, the number of the first port 12 and the number of the second port 13 may both be three, four, five, or more. The first ports 12 and the second ports 13 may be of a regular shape, such as a circular shape or a polygonal shape, or of an irregular shape. The shape of each of the first ports 12 and the second ports 13 may be the same or different. In an embodiment of the present application, to facilitate the formation and manufacturing of the first port 12 and the second port 13 and/or the connection thereof with a common tube, the first port 12 and the second port 13 are of a circular shape, and the first communication groove 14 and the second communication groove 15 have a circular cross section. For ease of manufacture, the size of the first port 12 may be the same as the size of the second port 13.

In some embodiments, the first communication groove 14 includes a main communication groove 141 and a plurality of branch communication grooves 142. The plurality of branch communication grooves 142 are all in communication with the main communication groove 141, and the plurality of branch communication grooves 142 are arranged in a one-to-one correspondence with the plurality of first ports 12; the plurality of first ports 12 are in communication with the common port 11 through the main communication groove 141 and the plurality of branch communication grooves 142.

In this way, the fluid can enter through the common port 11 and exit from the plurality of first ports 12 via the main communication groove 141 and the plurality of branch communication grooves 142, thereby achieving the diversion of a single fluid flow.

Specifically, the number of the main communication groove 141 is one, and the number of the branch communication grooves 142 may be three, four, five, six, or more. For example, the common port 11 is in communication with the main communication groove 141, the number of the first ports 12 is four, the number of the branch communication grooves 142 is four, one end of each of the four branch communication grooves 142 is in communication with one of the four first ports 12 in a one-to-one correspondence, and the other end is in communication with the main communication groove 141.

The main communication groove 141 and the branch communication grooves 142 may be of a bent shape, a curved shape, or the like, and the specific shapes of the main communication groove 141 and the branch communication grooves 142 are not limited herein. In one embodiment, the main communication groove 141 and the branch communication grooves 142 are linear grooves, which allows the distance of the fluid from the common port 11 to the first port 12 to be relatively short, such that the fluid inlet time can be shortened, thereby improving the sequencing efficiency.

Referring to FIG. 1, in some embodiments, the first communication groove 14 includes a main communication groove 141 and a plurality of sets of branch communication grooves 142. The plurality of sets of branch communication grooves 142 are in communication with the main communication groove 141, the plurality of sets of branch communication grooves 142 include a plurality of branch communication grooves 142, and the plurality of branch communication grooves 142 are arranged in a one-to-one correspondence with the plurality of first ports 12; the plurality of first ports 12 are in communication with the common port 11 through the main communication groove 141 and the plurality of branch communication grooves 142.

In this way, the fluid can enter through the common port 11 and exit from the plurality of first ports 12 via the main communication groove 141 and the plurality of sets of branch communication grooves 142, thereby achieving the flow diversion of a single fluid.

Specifically, the number of the main communication groove 141 is one, and the number of the branch communication grooves 142 may be three, four, five, six, or more. For example, the common port 11 is in communication with the main communication groove 141; the number of the first ports 12 is six, and the number of the branch communication grooves 142 is three sets; each of the sets of branch communication grooves 142 includes two branch communication grooves 142, one end of each of the branch communication grooves 142 in the three sets is arranged in a one-to-one correspondence with the six first ports 12, and the other end is in communication with the main communication groove 141. Alternatively, the number of the branch communication grooves 142 may be two sets; each of the sets of the branch communication grooves 142 includes three branch communication grooves 142, one end of each of the branch communication grooves 142 in the two sets is arranged in a one-to-one correspondence with the six first ports 12, and the other end is in communication with the main communication groove 141. The main communication groove 141 and the branch communication grooves 142 may be of a bent shape, a curved shape, or the like, and the specific shapes of the main communication groove 141 and the branch communication grooves 142 are not limited herein.

Referring to FIG. 1, in some embodiments, the plurality of sets of branch communication grooves 142 include a first set of branch communication grooves 142 and a second set of branch communication grooves 142. The first set of branch communication grooves 142 is in communication with the main communication groove 141 through a first flow path 143; the second set of branch communication grooves 142 is in communication with the main communication groove 141 through a second flow path 144.

In this way, the fluid can enter from the main communication groove 141 to the first set of branch communication grooves 142 and the second set of branch communication grooves 142, thereby achieving the diversion of a single fluid flow.

Specifically, the first set of branch communication grooves 142 and the second set of branch communication grooves 142 may include the same number of branch communication grooves 142, or may include different numbers of branch communication grooves 142. For example, the first set of branch communication grooves 142 and the second set of branch communication groove 142 both include three branch communication grooves 142. For another example, the first set of branch communication grooves 142 includes two branch communication grooves 142, and the second set of branch communication grooves 142 includes three branch communication grooves 142. The first flow path 143 and the second flow path 144 may be communication grooves of a bent shape, a curved shape, or the like, which is not particularly limited herein. One end of the first flow path 143 is connected to the main communication groove 141, and the other end is in communication with the first set of branch communication grooves 142, and one end of each of the first set of branch communication grooves 142 distal to the first flow path 143 is in communication with the first port 12. Similarly, one end of the second flow path 144 is connected to the main communication groove 141, and the other end is in communication with the second set of branch communication grooves 142, and one end of each of the second set of branch communication grooves 142 distal to the second flow path 144 is in communication the first port 12.

In one embodiment, the first set of branch communication grooves 142 and the second set of branch communication grooves 142 both include two branch communication grooves 142, the first set of branch communication grooves 142 and the second set of branch communication grooves 142 are symmetrically arranged about the main communication groove 141, and the two branch communication grooves 142 of the first set of branch communication grooves 142 are symmetrically arranged, and the two branch communication grooves 142 of the second set of branch communication grooves 142 are symmetrically arranged, which allows flow distances of the fluid from the common port 11 to the four first ports 12 to be equal, so as to allow the fluid to simultaneously reach the first ports 12 at the same flow velocity, thereby maintaining the fluid inflow consistency and improving the sequencing efficiency.

Referring to FIG. 1, in some embodiments, the main communication groove 141 is provided with a fluid inlet end, each of the branch communication grooves 142 is provided with a fluid outlet end, the fluid inlet end is in communication with the common port 11, and the fluid outlet ends are in communication with the first ports 12.

In this way, the fluid is facilitated to enter the flow path selection valve 10 from the common port 11 via the fluid inlet end, and exit the flow path selection valve 10 from the first ports 12 via the fluid outlet ends, thereby achieving the inflow and outflow of the fluid.

Specifically, the fluid inlet end may be located at an end of the main communication groove 141 distal to the branch communication groove 142, or may be located at the middle position of the main communication groove 141 along the length direction, and the fluid outlet ends are located at ends of the branch communication grooves 142 distal to the main communication groove 141.

Referring to FIG. 2, in some embodiments, a plurality of second communication grooves 15 are provided. Each of the second communication grooves 15 is provided with two ends, where one end of each of the second communication grooves 15 is in communication with one of the first ports 12, and the other end is in communication with one of the second ports 13.

In this way, the fluid from each of the first ports 12 can exit the corresponding second port 13 via the corresponding second communication groove 15, thereby achieving the independent control of a plurality of fluids, which avoids the cross contamination among the plurality of fluids, and improves the sequencing quality and sequencing efficiency.

Specifically, the second communication groove 15 may be a linear groove, and the length of the second communication groove 15 equals to the distance between the first port 12 and the corresponding second port 13. The first communication groove 14 may be arranged apart from the second communication groove 15, and the plurality of second communication grooves 15 may be arranged apart from each other.

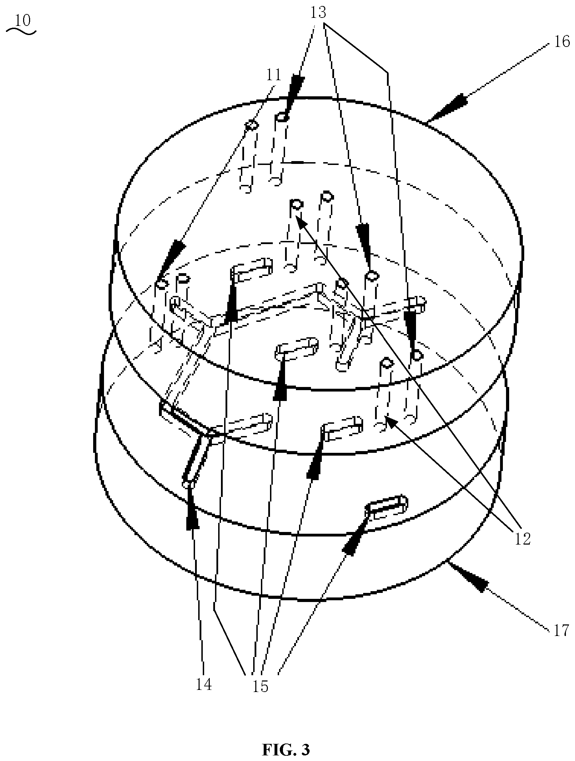

Referring to FIG. 3, in some embodiments, the flow path selection valve 10 includes a stator 16 and a rotor 17 disposed opposite to the stator 16. The stator 16 is provided with the common port 11, the plurality of first ports 12, and the plurality of second ports 13. The rotor 17 is provided with the first communication groove 14 and the second communication grooves 15.

In this way, by rotating the rotor 17, the first communication groove 14 can allow the first port 12 to be in communication with the common port 11, or the second communication groove 15 can allow the first port 12 to be in communication with the second port 13, such that the control over the switching of fluid flow paths can be achieved.

Specifically, the stator 16 and the rotor 17 may be coaxially arranged, or in other words, the central axis of the stator 16 and the central axis of the rotor 17 coincide. It can be understood that the rotor 17 may rotate relative to the stator 16. Further, the rotor 17 rotates relative to the stator 16 between the first valve position and the second valve position. In the case that the rotor 17 is in the first valve position relative to the stator 16, the first port 12 is in communication with the common port 11; in the case that the rotor 17 is in the second valve position relative to the stator 16, the first port 12 is in communication with the second port 13; in the case that the rotor 17 is between the first valve position and the second valve position relative to the stator 16, the common port 11, the first port 12, and the second port 13 are separated from one another. Unless otherwise stated, reference herein to a plurality of ports being "separated" means that the plurality of ports cannot be in communication with one another, or that the fluid cannot enter from one or more designated ports and exit from one or more other designated ports.

The common port 11, the first port 12, and the second port 13 may be circular through holes that penetrate the stator 16 in the thickness direction of the stator 16. The first communication groove 14 and the second communication groove 15 may be of a bent shape, a curved shape, or the like, and the specific shapes of the first communication groove 14 and the second communication groove 15 are not limited herein.

Referring to FIG. 1, in some embodiments, the number of the common ports 11 is plural. In the case that the flow path selection valve 10 is in the first valve position, each of the common ports 11 is in communication with the plurality of first ports 12 through the first communication groove 14.

In this way, the fluids from the plurality of common ports 11 can flow simultaneously to the first ports 12 through the first communication groove 14, which reduces the fluid inlet time, thereby improving the fluid inlet efficiency.

Specifically, the number of the common ports 11 may be two. One of the common ports 11 is in communication with the sample under test, and the other common port 11 is in communication with the reagent, allowing the sample under test and the reagent to flow simultaneously in the first communication groove 14. The first port 12 may be in communication with one of the common ports 11, or may be simultaneously in communication with each of the common ports 11.

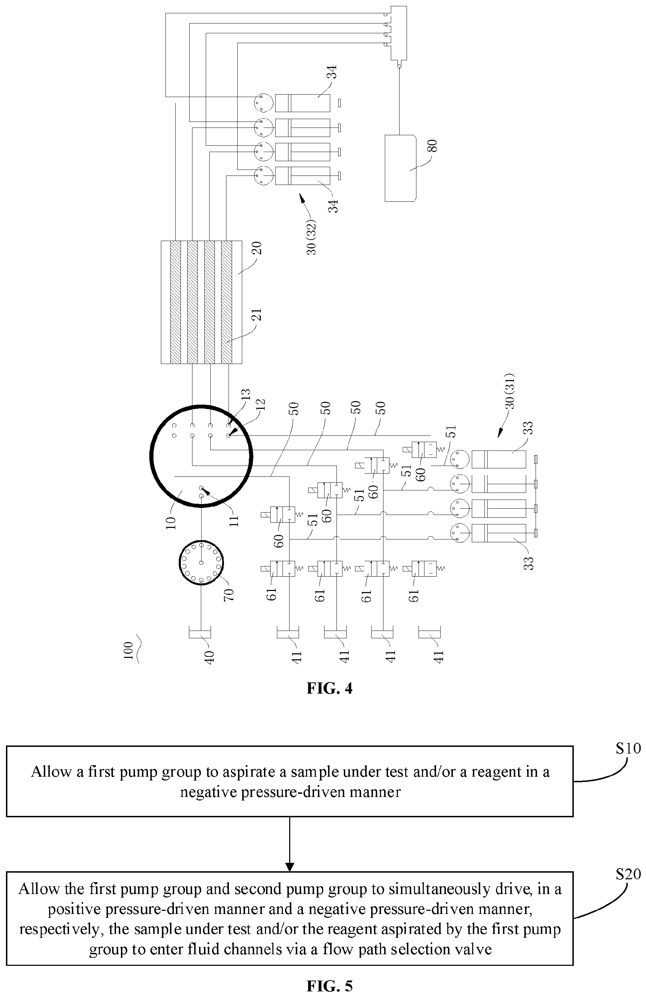

Referring to FIG. 4, the embodiments of the present application provide a fluidic system 100. The fluidic system includes a flow cell 20, a flow path selection valve 10, and a power assembly 30. The flow cell 20 includes a plurality of fluid channels 21, and the fluid channels 21 are configured to carry the sample under test and/or the reagent. The flow path selection valve 10 is disposed upstream of the flow cell 20, and a plurality of second ports 13 of the flow path selection valve 10 are in communication with and arranged in a one-to-one correspondence with the plurality of fluid channels 21. The power assembly 30 is configured to provide power to allow the sample under test and/or the reagent to enter the fluid channels 21 via the flow path selection valve 10.

In this way, the flow path selection valve 10 and the power assembly 30 can allow the sample under test and/or the reagent to enter the plurality of fluid channels 21, thereby achieving sequence determination of the sample under test.

Specifically, the flow cell 20 is configured to provide a place for biochemical reaction during sequencing. The flow cell 20 may also be referred to as a chip, and the flow cell 20 may be detachably connected to the flow path selection valve 10. The fluid channel 21 is provided with a space for accommodating fluid and can accommodate the sample under test and the reagent, thereby allowing the sample under test to undergo a biochemical reaction with the reagent. The sample under test may be fixed on the inner surface of the fluid channel 21. The plurality of fluid channels 21 in the flow cell 20 are arranged in parallel. The number of fluid channels 21 may be three, four, five, six, etc., and the size of each of the fluid channels 21 may be the same or different. In some embodiments, the fluid channel 21 has a non-circular cross-section or an approximately rectangular cross-section. The width of the fluid channel 21 is at least 2 mm, approximately 4 mm, or approximately 7 mm, and the height of the fluid channel 21 may be at least 0.8 mm or approximately 1.2 mm.

The sample under test includes at least one nucleic acid molecule, and the reagent includes a polymerase and at least one nucleotide molecule. The polymerase is used to bind the nucleotide molecule to the nucleic acid molecule. The base types may be the same or different among the plurality of nucleotide molecules. The nucleotide molecule is provided with a blocking group for blocking the binding of more than one nucleotide to the nucleic acid molecule during the single base extension reaction and the blocking group is provided with an optically detectable label that allows the reaction between the nucleotide molecule and the nucleic acid molecule to be detected, thereby achieving sequence determination of the nucleic acid molecule.

Each of the fluid channels 21 is provided with an inlet and an outlet, each of the second ports 13 of the flow path selection valve 10 is connected to the inlet of one fluid channel 21, and the power assembly 30 drives the sample under test and/or the reagent from the second ports 13 to enter the fluid channels 21 through the inlets. The power assembly 30 includes, but is not limited to, a device capable of providing power and pipelines for transmitting power. The power assembly 30 may be connected to the fluid channels 21 through conduits.

Referring to FIG. 4, in some embodiments, the fluidic system 100 includes a first reservoir 40. The first reservoir 40 is in communication with the common port 11 of the flow path selection valve 10, and the first reservoir 40 is configured to store the sample under test and/or the reagent.

In this way, the sample under test and/or the reagent in the first reservoir 40 can enter the flow path selection valve 10 through the common port 11, and subsequently flow to the fluid channels 21 of the flow cell 20, thereby achieving sequence determination of the sample under test.

Specifically, the first reservoir 40 may be a conduit or a reagent bottle. The number of the first reservoir 40 may be one or two, or plural, and each of the first reservoirs 40 stores one sample under test or one reagent. Different first reservoirs 40 may have the same or varying volumes. For example, each of the first reservoirs 40 may have the same volume, or each of the first reservoirs 40 may have a different volume, which may be determined based on the desired storage volume of the fluid stored in each of the first reservoirs 40. The first reservoir 40 may be in communication with the common port 11 through a conduit.

Referring to FIG. 4, in some embodiments, the power assembly 30 includes a first pump group 31 and a second pump group 32. The first pump group 31 is disposed upstream of the flow cell 20 and is in communication with the first ports 12 of the flow path selection valve 10, the first pump group 31 is in selective communication with the flow path selection valve 10 and the first reservoir 40, and the first pump group 31 is configured to aspirate the sample under test and/or the reagent in a negative pressure-driven manner, and drive, in a positive pressure-driven manner, the sample under test and/or the reagent to enter the fluid channels 21. The second pump group 32 is disposed downstream of the flow cell 20, and the second pump group 32 is configured to drive the sample under test and/or the reagent aspirated by the first pump group 31 to enter the fluid channels 21 in a negative pressure-driven manner.

In some embodiments, the first pump group 31 is configured to first aspirate the sample under test and/or the reagent in a negative pressure-driven manner, and then drive, in a positive pressure-driven manner, the aspirated sample under test and/or reagent to enter the fluid channels 21, simultaneously in combination with the second pump group 32 in a negative pressure-driven manner.

In this way, by allowing the first pump group 31 to first aspirate the sample under test and/or the reagent in a negative pressure-driven manner, and then provide power to drive, in a positive pressure-driven manner, the sample under test and/or the reagent to enter the fluid channels 21, simultaneously in combination with the second pump group 32, the increase in the flow velocity of the sample under test and/or the reagent is facilitated. Meanwhile, this approach avoids the adverse effects of susceptibility to the precipitation of bubbles in the sample under test and/or the reagent or the formation of bubbles due to the introduction of external gas into the sample under test and/or the reagent when using a single negative pressure-driven manner to increase the flow velocity, and prevents damage to the flow cell 20 resulting from excessive pressure in the flow cell 20 caused by using a single positive pressure-driven manner to increase the flow velocity.

Specifically, the first pump group 31 and the second pump group 32 may be a peristaltic pump, a plunger pump, a syringe pump, a gear pump, a diaphragm pump, or the like. The first pump group 31 and the second pump group 32 may provide negative pressure or positive pressure. The first pump group 31 includes a plurality of first single pumps 33, and the plurality of first single pumps 33 are in a one-to-one correspondence with the plurality of first ports 12. The second pump group 32 includes a plurality of second single pumps 34, and the plurality of second single pumps 34 are in a one-to-one correspondence with the outlets of the plurality of fluid channels 21.

Referring to FIG. 4, in some embodiments, the fluidic system 100 includes a plurality of first buffer regions 50. Each of the first buffer region 50 is in communication with one of the first ports 12 and one first single pump 33. In the case that the flow path selection valve 10 is in the first valve position, the first pump group 31 is configured to aspirate a single sample under test and/or reagent from the first reservoir 40 in a negative pressure-driven manner, and pump the single sample under test and/or reagent to each of the first buffer regions 50.

In this way, the flow path selection valve 10 can divide the single sample under test and/or reagent entering from the common port 11 into a plurality of portions, allowing the first reservoir 40 to be in communication with the common port 11 through a common conduit. Compared with driving, in a single positive pressure-driven manner, the sample under test and/or the reagent to enter the flow path selection valve 10 from the first reservoir 40 via a plurality of conduits, this configuration reduces the number of common conduits and reduces the reagent consumption. In addition, the first buffer region 50 can store the sample under test and/or the reagent from the first reservoir 40, so as to facilitate the switching of the first pump group 31 from the negative pressure-driven manner to the positive pressure-driven manner.

Specifically, the first buffer region 50 may be a conduit or a fluid storage bottle. The first pump group 31 provides negative pressure to drive the single sample under test and/or reagent in the first reservoir 40 to enter the flow path selection valve 10 from the common port 11 and then enter the corresponding first buffer region 50 from each of the first ports 12.

Referring to FIG. 4, in some embodiments, in the case that the flow path selection valve 10 is in the second valve position, the first pump group 31 is configured to pump the sample under test and/or the reagent in each of the first buffer regions 50 to the corresponding fluid channel 21 in a positive pressure-driven manner; and/or the second pump group 32 is configured to pump the sample under test and/or the reagent in each of the first buffer regions 50 to the corresponding fluid channel 21 in a negative pressure-driven manner.

In this way, the first pump group 31 first operates to pump the sample under test and/or the reagent in the first reservoir 40 into each of the first buffer regions 50 via the flow path selection valve 10 in a negative pressure-driven manner. Since the sample under test and/or the reagent is pumped into each of the first buffer regions 50 via the flow path selection valve 10 without passing through the fluid channel 21, the sample under test and/or the reagent is not influenced by the flow resistance in the fluid channel 21. Under the same negative pressure provided, compared with the flow velocity achieved when the negative pressure generated by the second pump group 32 is used to drive the sample under test and/or the reagent to directly enter the fluid channels 21 via the flow path selection valve 10, a higher flow velocity may be achieved when the negative pressure generated by first pump group 31 is used to drive the sample under test and/or the reagent into each of the first buffer regions 50 via the flow path selection valve 10. Then, the first pump group 31 operates in a positive pressure-driven manner and/or the second pump group 32 operates in a negative pressure-driven manner to pump the sample under test and/or the reagent in each of the first buffer regions 50 to the corresponding fluid channel 21. Therefore, by allowing the first pump group 31 to first operate in a negative pressure-driven manner to pump the sample under test and/or the reagent in the first reservoir 40 into each of the first buffer regions 50 via the flow path selection valve 10 and then allowing the first pump group 31 to operate in a positive pressure-driven manner and/or the second pump group 32 to operate in a negative pressure-driven manner to pump the sample under test and/or the reagent in each of the first buffer regions 50 to the corresponding fluid channel 21, the increase in the flow velocity of the sample under test and/or the reagent is facilitated, thereby saving time and improving efficiency. Specifically, the situation may be that the first pump group 31 provides positive pressure to drive the sample under test and/or the reagent in each of the first buffer regions 50 to enter the flow path selection valve 10 from the corresponding first port 12 and then flow into the corresponding fluid channel 21 from the corresponding second port 13; or the second pump group 32 provides negative pressure to drive the sample under test and/or the reagent in each of the first buffer regions 50 to enter the flow path selection valve 10 from the corresponding first port 12 and then flow into the corresponding fluid channel 21 from the corresponding second port 13; or the first pump group 31 provides positive pressure and simultaneously the second pump group 32 provides negative pressure to drive the sample under test and/or the reagent in each of the first buffer regions 50 to enter the flow path selection valve 10 from the corresponding first port 12 and then flow into the corresponding fluid channel 21 from the corresponding second port 13.

Referring to FIG. 4, in some embodiments, the fluidic system 100 includes a plurality of first switching valves 60. The first switching valves 60 are disposed between the first pump group 31 and the flow path selection valve 10 and are configured to control the communication and disconnection between the first pump group 31 and the flow path selection valve 10.

In this way, the first switching valves 60 can control the communication and disconnection between the first pump group 31 and the flow path selection valve 10, thereby controlling the fluid flow.

Specifically, the first switching valves 60 may be solenoid valves. Each of the first switching valves 60 is connected to one first single pump 33 and one first port 12, and each of the first buffer regions 50 is in communication with one first port 12 and one first switching valve 60. In the case that the first switching valves 60 are energized, the first pump group 31 is in communication with the flow path selection valve 10, and the first pump group 31 can drive a single sample under test and/or reagent in the first reservoir 40 to enter a plurality of first buffer regions 50 through the flow path selection valve 10, or drive the sample under test and/or the reagent in each first buffer region 50 to enter the fluid channel 21 through the flow path selection valve 10; in the case that the solenoid valves are de-energized, the communication between the first pump group 31 and the flow path selection valve 10 is cut off.

Referring to FIG. 4, in some embodiments, the fluidic system 100 includes a plurality of second buffer regions 51 and a plurality of second reservoirs 41. Each of the second buffer regions 51 is in communication with one first single pump 33 and one second switching valve 61, and the first pump group 31 is configured to aspirate a plurality of samples under test from the plurality of second reservoirs 41 in a negative pressure-driven manner, and pump each of the samples under test to one of the second buffer regions 51 corresponding thereto.

In this way, the second buffer regions 51 can store the sample under test from the second reservoirs 41, so as to facilitate the switching of the first pump group 31 from the negative pressure-driven manner to the positive pressure-driven manner. The sample under test in the second reservoir 41 can enter the flow path selection valve 10 through the second switching valves 61 and the first switching valves 60, and subsequently flow to the fluid channels 21 of the flow cell 20, thereby achieving sequence determination of the sample under test.

Specifically, the second buffer region 51 may be a conduit or a fluid storage bottle. Each of the second buffer regions 51 is in communication with one first switching valve 60 and one first single pump 33. The first pump group 31 provides negative pressure to drive the plurality of samples under test in the plurality of second reservoirs 41 to enter the plurality of second buffer regions 51 through the second switching valves 61.

The second reservoir 41 may be a conduit or a reagent bottle. The number of the second reservoir 41 may be one, two, or plural, and each of the second reservoirs 41 stores one sample under test. Different second reservoirs 41 may have the same or varying volumes. For example, each of the second reservoirs 41 may have the same volume, or each of the second reservoirs 41 may have a different volume, which may be determined based on the desired storage volume of the sample under test stored in each of the second reservoirs 41. The second reservoirs 41 may be in communication with the second switching valves 61 through conduits.

Referring to FIG. 4, in some embodiments, in the case that the flow path selection valve 10 is in the second valve position, the first pump group 31 is configured to pump the sample under test in each of the second buffer regions 51 to the corresponding fluid channel 21 in a positive pressure-driven manner; and/or the second pump group 32 is configured to pump the sample under test in each of the second buffer regions 51 to the corresponding fluid channel 21 in a negative pressure-driven manner.

In this way, the first pump group 31 and/or the second pump group 32 can drive the sample under test in each of the second buffer regions 51 to enter the corresponding fluid channel 21, thereby achieving simultaneous sample introduction of the plurality of samples under test.

Specifically, the situation may be that the first pump group 31 provides positive pressure to drive the sample under test in each of the second buffer regions 51 to enter the flow path selection valve 10 from the corresponding first port 12 via the corresponding first switching valve 60 and then flow into the corresponding fluid channel 21 from the corresponding second port 13; or the second pump group 32 provides negative pressure to drive the sample under test in each of the second buffer regions 51 to enter the flow path selection valve 10 from the corresponding first port 12 via the corresponding first switching valve 60 and then flow into the corresponding fluid channel 21 from the corresponding second port 13; or the first pump group 31 provides positive pressure and simultaneously the second pump group 32 provides negative pressure to drive the sample under test in each of the second buffer regions 51 to enter the flow path selection valve 10 from the corresponding first port 12 via the corresponding first switching valve 60 and then flow into the corresponding fluid channel 21 from the corresponding second port 13.

Referring to FIG. 4, in some embodiments, the fluidic system 100 includes a plurality of second switching valves 61. Each of the second switching valves 61 is disposed between the second reservoir 41 and the second buffer region 51 corresponding thereto and is configured to control the communication and disconnection between the second reservoir 41 and the second buffer region 51. In this way, the second switching valve 61 can control the communication and disconnection between the second reservoir 41 and the second buffer region 51, so as to control the flow of the sample under test.

Specifically, the second switching valve 61 may be a solenoid valve. Each of the second switching valves 61 is connected to one first single pump 33 and one sample under test in the second reservoir 41. In the case that the second switching valve 61 is energized, the second reservoir 41 is in communication with the second buffer region 51, and the first pump group 31 can drive the sample under test in each second reservoir 41 to enter the corresponding second buffer region 51; in the case that the solenoid valve is de-energized, the communication between the second reservoir 41 and the second buffer region 51 is cut off. The second reservoir 41 is connected to the second switching valve 61 in a manner including, but not limited to, barbed fittings, threaded joints, and the like.

Referring to FIG. 4, in some embodiments, the fluidic system 100 includes a rotary valve 70. The rotary valve 70 is in communication with the first reservoir 40 and the common port 11 of the flow path selection valve 10.

In this way, the rotary valve 70 can be in selective communication with the first reservoir 40 and the common port 11, thereby achieving the switching of the sample under test or the reagent as well as the switching between different reagents.

Specifically, the rotary valve 70 may rotate in its own axial direction to allow the common port 11 to be in communication with different first reservoirs 40, facilitating the entry of different samples under test or reagents into the flow path selection valve 10. The first reservoir 40 is connected to the rotary valve 70 in a manner including, but not limited to, pipeline, barbed fittings, threaded joints, and the like.

In one embodiment, the fluidic system 100 includes a waste fluid bottle 80. The waste fluid bottle 80 is connected to the second pump group 32, and the waste fluid bottle 80 is configured to store and discharge all or a portion of the waste fluid of the fluidic system 100. The second pump group 32 can aspirate the waste fluid from each of the fluid channels 21 in a negative pressure-driven manner and pump the aspirated waste fluid to the waste fluid bottle 80 in a positive pressure-driven manner.

The embodiments of the present application provide a method for controlling fluid flow for use in the fluidic system 100 according to any one of the above embodiments. The method includes:
allowing the power assembly 30 to drive, in both a positive pressure-driven manner and a negative pressure-driven manner, fluid including a sample under test and/or a reagent to enter a fluid channel 21 via the flow path selection valve 10.

In this way, by allowing the power assembly 30 to drive the fluid to enter the fluid channel 21 in both a positive pressure-driven manner and a negative pressure-driven manner, the increase in the flow velocity of the sample under test and/or the reagent is facilitated. Meanwhile, this approach can avoid the susceptibility to the precipitation of bubbles in the fluid or the formation of bubbles due to the introduction of external gas into the fluid when using a single negative pressure-driven manner to increase the flow velocity, and also prevent damage to the flow cell 20 resulting from excessive pressure in the flow cell 20 caused by using a single positive pressure-driven manner to increase the flow velocity.

Specifically, the power assembly 30 may drive, in both a positive pressure-driven manner and a negative pressure-driven manner, the sample under test to enter the fluid channel 21 via the flow path selection valve 10; or the power assembly may drive, in both a positive pressure-driven manner and a negative pressure-driven manner, the reagent to enter the fluid channel 21 via the flow path selection valve 10; or the power assembly may drive, in both a positive pressure-driven manner and a negative pressure-driven manner, the sample under test and the reagent to enter the fluid channel 21 via the flow path selection valve 10.

Referring to FIG. 5, in some embodiments, allowing the power assembly 30 to drive, in both a positive pressure-driven manner and a negative pressure-driven manner, the sample under test and/or the reagent to enter the fluid channel 21 via the flow path selection valve 10 includes:
S10, allowing a first pump group 31 to aspirate the sample under test and/or the reagent in a negative pressure-driven manner; and
S20, allowing the first pump group 31 and the second pump group 32 to simultaneously drive, in a positive pressure-driven manner and a negative pressure-driven manner, respectively, the sample under test and/or the reagent aspirated by the first pump group 31 to enter the fluid channel 21 via the flow path selection valve 10.

In this way, by first allowing the first pump group 31 to aspirate the sample under test and/or the reagent in a negative pressure-driven manner and then allowing the first pump group 31 to provide positive pressure and the second pump group 32 to provide negative pressure simultaneously, the increase in the flow velocity of the fluid is facilitated. In the case that the positive pressure provided by the first pump group 31 and the negative pressure provided by the second pump group 32 are constant, the absolute value of the total pressure difference in the fluidic system remains unchanged, the pressure distribution in the fluidic system 100 gradually changes from the positive pressure to the negative pressure along the flow direction of the sample under test and/or the reagent in the flow cell 20, which reduces the pressure borne by the flow cell 20. Compared with using a single positive pressure-driven manner or a single negative pressure-driven manner, this approach avoids the precipitation of bubbles in the fluid or the formation of bubbles due to the introduction of external gas into the fluid, and also prevents damage to the flow cell 20 caused by excessive pressure in the flow cell 20. Therefore, the flow cell 20 with lower cost and simpler structure can be selected, thereby reducing the sequencing cost.

Specifically, the situation may be that after the first pump group 31 aspirates the sample under test in a negative pressure-driven manner, the first pump group 31 and the second pump group 32 simultaneously drive, in a positive pressure-driven manner and a negative pressure-driven manner, respectively, the sample under test aspirated by the first pump group 31 to enter the fluid channel 21 via the flow path selection valve 10; or after the first pump group 31 aspirates the reagent in a negative pressure-driven manner, the first pump group 31 and the second pump group 32 simultaneously drive, in a positive pressure-driven manner and a negative pressure-driven manner, respectively, the reagent aspirated by the first pump group 31 to enter the fluid channel 21 via the flow path selection valve 10; or after the first pump group 31 aspirates the sample under test and the reagent in a negative pressure-driven manner, the first pump group 31 and the second pump group 32 simultaneously drive, in a positive pressure-driven manner and a negative pressure-driven manner, respectively, the sample under test and the reagent aspirated by the first pump group 31 to enter the fluid channel 21 via the flow path selection valve 10.

In some embodiments, the method includes:
in the case that the flow path selection valve 10 is in the first valve position, allowing the first pump group 31 to aspirate a single sample under test and/or reagent from the first reservoir 40 in a negative pressure-driven manner, and pump the single sample under test and/or reagent to each of the first buffer regions 50.

In this way, the flow path selection valve 10 can divide the single sample under test and/or reagent entering from the common port 11 into a plurality of portions, allowing the first reservoir 40 to be in communication with the common port 11 through a common conduit. Compared with driving, in a single positive pressure-driven manner, the sample under test and/or the reagent to enter the flow path selection valve 10 from the first reservoir 40 via a plurality of common conduits, this configuration reduces the number of common conduits and reduces the reagent consumption. In addition, the first buffer region 50 can store the sample under test and/or the reagent from the first reservoir 40, so as to facilitate the switching of the first pump group 31 from the negative pressure-driven manner to the positive pressure-driven manner.

Specifically, the first pump group 31 drives, in a negative pressure-driven manner, the single sample under test and/or reagent in the first reservoir 40 to enter the flow path selection valve 10 via the rotary valve 70 from the common port 11, and then enter the corresponding first buffer region 50 from each of the first ports 12.

In some embodiments, the method includes:
in the case that the flow path selection valve 10 is in the second valve position, allowing the first pump group 31 and the second pump group 32 to simultaneously pump the sample under test and/or the reagent in each of the first buffer regions 50 to the corresponding fluid channel 21 via the flow path selection valve 10 in a positive pressure-driven manner and a negative pressure-driven manner, respectively.

In this way, the single sample under test and/or reagent can be pumped to the plurality of fluid channels 21, such that the single sample under test can be simultaneously sequenced in the plurality of fluid channels 21, thereby improving the sequencing efficiency of the fluidic system 100. In addition, by allowing the first pump group 31 to provide positive pressure and the second pump group 32 to provide negative pressure simultaneously, the increase in the flow velocity of the fluid is facilitated. Meanwhile, this approach avoids the susceptibility to the precipitation of bubbles in the fluid or the formation of bubbles due to the introduction of external gas into the fluid when using a single negative pressure-driven manner to increase the flow velocity, and also prevent damage to the flow cell 20 resulting from excessive pressure in the flow cell 20 caused by using a single positive pressure-driven manner to increase the flow velocity.

Specifically, the first pump group 31 and the second pump group 32 simultaneously drive, in a positive pressure-driven manner and a negative pressure-driven manner, respectively, the sample under test and/or the reagent in each of the first buffer regions 50 to enter the flow path selection valve 10 from the corresponding first port 12 and then enter the corresponding fluid channel 21 from the corresponding second port 13 via the inlet of each of the fluid channels 21.

In some embodiments, the method includes:
allowing the first pump group 31 to aspirate a plurality of samples under test from a plurality of second reservoirs 41 in a negative pressure-driven manner, and pump each of the samples under test to one of the second buffer regions 51 corresponding thereto.

In this way, the second buffer regions 51 can store the plurality of samples under test from the plurality of second reservoirs 41, so as to facilitate the switching of the first pump group 31 from the negative pressure-driven manner to the positive pressure-driven manner.

Specifically, the first pump group 31 drives, in a negative pressure-driven manner, each of the plurality of samples under test from the plurality of second reservoirs 41 to enter the corresponding second buffer region 51 via a second switching valve 61.

In some embodiments, the method includes:
in the case that the flow path selection valve 10 is in the second valve position, allowing the first pump group 31 and the second pump group 32 to simultaneously pump, in a positive pressure-driven manner and a negative pressure-driven manner, respectively, the sample under test in each of the second buffer regions 51 to the corresponding fluid channel 21 via the flow path selection valve 10.

In this way, each of the samples under test can be pumped to the corresponding fluid channel 21, such that different samples under test can be simultaneously sequenced, thereby improving the sequencing efficiency of the fluidic system 100. In addition, by allowing the first pump group 31 to provide positive pressure and the second pump group 32 to provide negative pressure simultaneously, the increase in the flow velocity of the fluid is facilitated. Meanwhile, this approach avoids the susceptibility to the precipitation of bubbles in the fluid or the formation of bubbles due to the introduction of external gas into the fluid when using a single negative pressure-driven manner to increase the flow velocity, and also prevent damage to the flow cell 20 resulting from excessive pressure in the flow cell 20 caused by using a single positive pressure-driven manner to increase the flow velocity.

Specifically, the first pump group 31 and the second pump group 32 simultaneously drive, in a positive pressure-driven manner and a negative pressure-driven manner, respectively, the sample under test in each of the second buffer regions 51 to enter the flow path selection valve 10 from the corresponding first port 12 via a corresponding first switching valve 60, and then enter the corresponding fluid channel 21 from the corresponding second port 13 via the inlet of each of the fluid channels 21.

The embodiments of the present application provide a computer storage medium. A computer program, when run by a processor, causes the processor to implement the method according to any one of the above embodiments.

Specifically, in one embodiment, the processor may be a central processing unit (CPU). The processor may also be other general-purpose processors, digital signal processors (DSP), application specific integrated circuits (ASIC), field-programmable gate arrays (FPGA), or other chips such as programmable logic devices, discrete gates, transistor logic devices, discrete hardware components, or a combination thereof.

The computer program can be stored in a memory. The memory, as a non-transitory computer-readable storage medium, can be configured to store non-transitory software programs, non-transitory computer-executable programs, and modules, such as program instructions/modules corresponding to the methods in the aforementioned method embodiments. The processor, by executing non-transitory software programs, instructions, and modules stored in the memory, performs various functional applications and data processing of the processor, thereby implementing the control method in the aforementioned method embodiments.

The storage medium may include but is not limited to various media capable of storing computer programs, such as a U disk, a read-only memory (ROM), a random access memory (RAM), a portable hard disk, a magnetic disk, or an optical disk.

In the description of the specification, references to the terms such as "an embodiment", "some embodiments", "schematic embodiments", "examples", "specific examples", or "some examples" mean that the specific feature, structure, material, or characteristic described in connection with the embodiment or example is included in at least one embodiment or example of the present disclosure. In the specification, the schematic description of the aforementioned terms does not necessarily refer to the same embodiment or example. Moreover, the specific feature, structure, material, or characteristic described may be combined in any one or more embodiments or examples in an appropriate manner.

Although the embodiments of the present disclosure have been illustrated and described, it can be understood by those of ordinary skill in the art that various changes, modifications, replacements, and variations can be made to these embodiments without departing from the principle and purpose of the present disclosure, and the scope of the present disclosure is defined by the claims and equivalents therefore.

## Claims

1. A flow path selection valve, wherein the flow path selection valve is configured to switch between a first valve position and a second valve position, and the flow path selection valve is provided with a common port, a plurality of first ports, a plurality of second ports, a first communication groove, and second communication grooves; in a case that the flow path selection valve is in the first valve position, the plurality of first ports are in communication with the common port through the first communication groove; in a case that the flow path selection valve is in the second valve position, the first ports are in communication with the second ports through the second communication grooves in a one-to-one correspondence.

2. The flow path selection valve according to claim 1, wherein
the first communication groove comprises a main communication groove and a plurality of branch communication grooves, wherein the plurality of branch communication grooves are all in communication with the main communication groove, and the plurality of branch communication grooves are arranged in a one-to-one correspondence with the plurality of first ports;
the plurality of first ports are in communication with the common port through the main communication groove and the plurality of branch communication grooves.

3. The flow path selection valve according to claim 1, wherein
the first communication groove comprises a main communication groove and a plurality of sets of branch communication grooves, wherein the plurality of sets of branch communication grooves are in communication with the main communication groove, the plurality of sets of branch communication grooves comprise a plurality of branch communication grooves, and the plurality of branch communication grooves are arranged in a one-to-one correspondence with the plurality of first ports;
the plurality of first ports are in communication with the common port through the main communication groove and the plurality of branch communication grooves.

4. The flow path selection valve according to claim 3, wherein
the plurality of sets of branch communication grooves comprise a first set of branch communication grooves and a second set of branch communication grooves;
the first set of branch communication grooves is in communication with the main communication groove through a first flow path;
the second set of branch communication grooves is in communication with the main communication groove through a second flow path.

5. The flow path selection valve according to any one of claims 2 to 4, wherein
the main communication groove is provided with a fluid inlet end, each of the branch communication grooves is provided with a fluid outlet end, the fluid inlet end is in communication with the common port, and the fluid outlet ends are in communication with the first ports;
optionally, a plurality of second communication grooves are provided, each of the second communication grooves has two ends, wherein one end of each second communication groove is in communication with one of the first ports, and the other end is in communication with one of the second ports;
optionally, the flow path selection valve comprises a stator and a rotor disposed opposite to the stator, wherein the stator is provided with the common port, the plurality of first ports, and the plurality of second ports, and the rotor is provided with the first communication groove and the second communication grooves;
optionally, the number of the common ports is plural; in the case that the flow path selection valve is in the first valve position, each of the common ports is in communication with the plurality of first ports through the first communication groove.

6. A fluidic system, comprising:
a flow cell, wherein the flow cell comprises a plurality of fluid channels, and the fluid channels are configured to carry a sample under test and/or a reagent;
the flow path selection valve according to any one of claims 1 to 8, wherein the flow path selection valve is disposed upstream of the flow cell, a plurality of second ports of the flow path selection valve are in communication with and arranged in a one-to-one correspondence with the plurality of fluid channels; and
a power assembly configured to provide power to drive the sample under test and/or the reagent to enter the fluid channels via the flow path selection valve.

7. The fluidic system according to claim 6, wherein
the fluidic system comprises a first reservoir, wherein the first reservoir is in communication with the common port of the flow path selection valve, and the first reservoir is configured to store the sample under test and/or the reagent;
the power assembly comprises a first pump group and a second pump group, wherein the first pump group is disposed upstream of the flow cell and is in communication with first ports of the flow path selection valve, the first pump group is in selective communication with the flow path selection valve and the first reservoir, and the first pump group is configured to aspirate the sample under test and/or the reagent in a negative pressure-driven manner, and drive, in a positive pressure-driven manner, the sample under test and/or the reagent to enter the fluid channels;
the second pump group is disposed downstream of the flow cell, and the second pump group is configured to drive, in a negative pressure-driven manner, the sample under test and/or the reagent aspirated by the first pump group to enter the fluid channels.

8. The fluidic system according to claim 7, wherein
the fluidic system comprises a plurality of first buffer regions, wherein each of the first buffer regions is in communication with one of the first ports and the first pump group, and in a case that the flow path selection valve is in a first valve position, the first pump group is configured to aspirate a single sample under test and/or a reagent from the first reservoir in a negative pressure-driven manner, and pump the single sample under test and/or the reagent to each of the first buffer regions;
in a case that the flow path selection valve is in a second valve position, the first pump group is configured to pump the sample under test and/or the reagent in each of the first buffer regions to the corresponding fluid channel in a positive pressure-driven manner; and/or
the second pump group is configured to pump the sample under test and/or the reagent in each of the first buffer regions to the corresponding fluid channel in a negative pressure-driven manner.

9. The fluidic system according to claim 8, wherein
the fluidic system comprises a plurality of first switching valves, wherein the first switching valves are disposed between the first pump group and the flow path selection valve and are configured to control communication and disconnection between the first pump group and the flow path selection valve.

10. The fluidic system according to claim 9, wherein
the fluidic system comprises a plurality of second buffer regions, a plurality of second switching valves and a plurality of second reservoirs, wherein each of the second buffer regions is in communication with the first pump group and one second switching valve, each of the second switching valves is disposed between the second reservoir and the second buffer region corresponding thereto and is configured to control communication and disconnection between the second reservoir and the second buffer region, and the first pump group is configured to aspirate a plurality of samples under test from the second reservoirs in a negative pressure-driven manner, and pump each of the samples under test to one of the second buffer regions corresponding thereto; in the case that the flow path selection valve is in the second valve position, the first pump group is configured to pump the sample under test in each of the second buffer regions to the corresponding fluid channel in a positive pressure-driven manner; and/or
the second pump group is configured to pump the sample under test in each of the second buffer regions to the corresponding fluid channel in a negative pressure-driven manner;
optionally, the fluidic system comprises a rotary valve, wherein the rotary valve is in communication with the first reservoir and the common port of the flow path selection valve.

11. A method for controlling fluid flow for use in the fluidic system according to any one of claims 6 to 10, wherein
the fluidic system comprises a flow cell, a flow path selection valve, and a power assembly, wherein the flow cell comprises a plurality of fluid channels, and the fluid channels are configured to carry a sample under test and/or a reagent; the flow path selection valve is disposed upstream of the flow cell, and a plurality of second ports of the flow path selection valve are in communication with and arranged in a one-to-one correspondence with the plurality of fluid channels;
the method comprises:
allowing the power assembly to drive, in both a positive pressure-driven manner and a negative pressure-driven manner, the sample under test and/or the reagent to enter the fluid channel via the flow path selection valve.

12. The method according to claim 11, wherein
the power assembly comprises a first pump group and a second pump group;
allowing the power assembly to drive, in both a positive pressure-driven manner and a negative pressure-driven manner, the sample under test and/or the reagent to enter the fluid channel via the flow path selection valve comprises:
allowing the first pump group to aspirate the sample under test and/or the reagent in a negative pressure-driven manner; and
allowing the first pump group and the second pump group to simultaneously drive, in a positive pressure-driven manner and a negative pressure-driven manner, respectively, the sample under test and/or the reagent aspirated by the first pump group to enter the fluid channel via the flow path selection valve.

13. The method according to claim 11 or 12, wherein
the fluidic system comprises a plurality of first buffer regions, and the power assembly comprises a first pump group and a second pump group;
the method comprises:
in a case that the flow path selection valve is in a first valve position, allowing the first pump group to aspirate a single sample under test and/or reagent from the first reservoir in a negative pressure-driven manner, and pumping the single sample under test and/or reagent to each of the first buffer regions;
in a case that the flow path selection valve is in a second valve position, allowing the first pump group and the second pump group to simultaneously pump the sample under test and/or the reagent in each of the first buffer regions to the corresponding fluid channel via the flow path selection valve in a positive pressure-driven manner and a negative pressure-driven manner, respectively.

14. The method according to claim 11 or 12, wherein
the fluidic system comprises a plurality of second buffer regions and a plurality of second reservoirs, and the power assembly comprises a first pump group and a second pump group;
the method comprises:
allowing the first pump group to aspirate a plurality of samples under test from the second reservoirs in a negative pressure-driven manner, and pumping each of the samples under test to one of the second buffer regions corresponding thereto;
in the case that the flow path selection valve is in the second valve position, allowing the first pump group and the second pump group to pump the sample under test in each of the second buffer regions to the corresponding fluid channel via the flow path selection valve in a positive pressure-driven manner and a negative pressure-driven manner, respectively.
optionally, the fluidic system comprises a rotary valve, wherein the rotary valve is in communication with the first reservoir and the common port of the flow path selection valve.

15. A computer storage medium, wherein
a computer program, when run by a processor, causes the processor to implement the method according to any one of claims 11 to 14.
